# FASCICULE DE BREVET EUROPEEN

(11) **EP 3 280 480 B1**
(45) Date de publication et mention de la délivrance du brevet: **05.06.2024**
(21) Numéro de dépôt: 16717310.3
(22) Date de dépôt: 08.04.2016
(51) Int. Cl.: A61M 25/00, A61M 1/16, A61M 1/36, A61M 25/04

(54) **DISPOSITIF DE CANULE, POUMON ARTIFICIEL**
KANÜLENVORRICHTUNG, KÜNSTLICHE LUNGE
CANNULA DEVICE, ARTIFICIAL LUNG

(30) Priorité: 09.04.2015 FR 1553080
(43) Date de publication de la demande: 14.02.2018
(73) Titulaire: Fondation Hôpital Saint Joseph, 75014 Paris (FR); Université Paris-Saclay, 91190 Gif-sur-Yvette (FR); Institut National de la Santé et de la Recherche Médicale, 75013 Paris (FR)
(72) Inventeur: OLAF, Mercier, 92260 Fontenay aux Roses (FR)
(74) Mandataire: Cabinet Camus Lebkiri
(86) Numéro de dépôt international: PCT/EP2016/057857
(87) Numéro de publication internationale: WO 2016/162543

(56) Documents cités:
- WO-A1-2015/009904
- US-A1- 2011 282 195
- US-A1- 2012 302 995
- US-A1- 2014 012 066

## Description

### DOMAINE TECHNIQUE DE L'INVENTION

La présente invention concerne un dispositif de canule pour la circulation du sang dans un poumon artificiel ainsi qu'un poumon artificiel comprenant ledit dispositif de canule.

L'invention trouve une application particulièrement intéressante pour la mise en place d'un poumon artificiel destiné à être utilisé par un patient pendant une longue durée.

### ARRIERE PLAN TECHNOLOGIQUE DE L'INVENTION

Il existe aujourd'hui des canules permettant d'offrir une solution de poumon artificiel. Ces canules permettent une circulation du sang dans un circuit fermé grâce à une technique d'oxygénation d'un volume de sang pauvre en oxygène prélevé d'un corps.

De telles canules sont actuellement utilisées, par exemple, avec un système de poumon artificiel de type ECMO, désignant « Extracorporel Membrane Oxygénation ». Un tel système permet d'offrir une solution d'oxygénation d'un volume de sang prélevé chez un patient et circulant par l'intermédiaire des canules au moyen d'une membrane.

Une première solution consiste à utiliser un couple de canules qui est introduit via les veines d'un patient. Le circuit ainsi formé est nommé VV car il est réalisé par voie veino-veineuse. Dans ce cas de figure, la circulation du sang se fait par les veines.

Une seconde solution consiste à utiliser un couple de canules dont l'une est introduite par voie veineuse et l'autre par voie artérielle. Ce circuit est nommé AV car il est réalisé par voie artério-veineuse. Pour cela des canules spécifiques sont adaptées au débit de sang à prélever et à injecter. On parle communément de canule artérielle et de canule veineuse.

Dans les deux cas, le sang extrait du système veineux est oxygéné à l'extérieur du corps. Dans l'ECMO VA, le sang ainsi ré-oxygéné est remis en circulation par le système artériel, alors que dans l'ECMO VV, l'opération se fait au travers du système veineux.

Un inconvénient de la première solution est la limitation de l'assistance cardiaque et pulmonaire d'un patient en sang oxygéné. L'avantage pratique d'introduire les canules par les voies veineuses trouve une limitation sur l'efficacité d'acheminement du sang oxygéné dans certains organes.

Un inconvénient de la seconde solution est la difficulté de mise en place et le maintien d'une canule d'injection dans une artère. Notamment, cette technique impose une mobilité réduite du patient car le débit du sang à injecter et les mouvements du fluide sanguin peuvent perturber la mise en place d'une canule et rendre instable son maintien.

WO 2015/009904 A1 décrit un système de circulation du sang avec un canule auto-expansible placée au niveau de la canule de drainage.

US 2014/012066 A1 décrit un système de canulation du coeur pouvant utiliser un maillage expansible pour l'isolation fluidique.

### RESUME L'INVENTION

L'invention est définie dans les revendications 1-19.

Un objet de l'invention concerne un dispositif de canule pour la circulation d'un fluide dans un poumon artificiel caractérisé en ce qu'il comprend :
- une première lumière, dite lumière d'aspiration, comprenant au moins un orifice permettant l'aspiration d'un volume du fluide ;
- une seconde lumière, dite lumière d'injection, comprenant au moins un orifice permettant l'injection d'un volume du fluide,
caractérisée en ce que la lumière d'injection et la lumière d'aspiration sont maintenues solidaire sur au moins une portion de leur longueur, lesdites lumières étant étanches l'une de l'autre, la lumière d'injection comportant un élément déformable maintenu sur une portion de son extrémité distale, ledit élément déformable comportant un premier agencement dit de « passage » et un second agencement dit « de maintien », le passage du premier agencement au second agencement s'effectuant par une modification du diamètre dudit élément déformable.

Un avantage est de permettre un maintien d'une canule pendant une longue durée.

Ceci est particulièrement avantageux lorsque le dispositif de canule est utilisé pour une application de poumon artificiel. Un autre avantage lorsqu'une lumière est introduite dans une cavité du coeur, par exemple, est de permettre une bonne stabilité et un maintien malgré les mouvements liés à la contraction du coeur.

La lumière d'injection comporte à son extrémité distale l'élément déformable maillé de sorte à permettre le passage d'un fluide entre ses mailles.

Selon un mode de réalisation, l'élément déformable maillé est extensible de sorte à permettre le passage d'un fluide entre ses mailles.

Un avantage est que l'élément déformable permet le passage d'un fluide, à cet effet les mailles peuvent être configurées de sorte à permettre un débit maximal.

Selon un mode de réalisation, l'élément déformable forme l'orifice distal de la lumière d'injection à son extrémité distale de sorte à permettre le passage d'un fluide entre ses mailles.

Selon un mode de réalisation, l'élément déformable prolonge l'orifice distal de la lumière d'injection à son extrémité distale de sorte à permettre le passage d'un fluide entre ses mailles.

Selon un mode de réalisation, l'élément déformable est extensible et rétractable. Selon un mode de réalisation :
▪ le premier agencement de l'élément déformable est obtenu par extension dudit élément déformable, le diamètre de l'élément déformable étant alors inférieur à un premier seuil ;
▪ le second agencement de l'élément déformable est obtenu dans sa forme de repos, le diamètre de l'élément déformable étant alors supérieur à un second seuil.

Un avantage est de permettre l'introduction de la lumière sous contrainte afin de relâcher la contrainte une fois le dispositif de canule en place. En conséquence, le dispositif de canule ne nécessite pas un maintien d'une contrainte sur l'élément déformable lors de son fonctionnement.

Selon un mode de réalisation, l'élément déformable est « à mémoire de forme ». Selon un mode de réalisation, l'élément déformable est un Stent.

Selon un mode de réalisation, l'élément déformable comprend une forme de repos réalisant un premier moyen de maintien du dispositif de canule, l'une des formes de repos pouvant être l'une des suivantes:
▪ Une forme de champignon ;
▪ Une forme d'assiette ;
▪ Une forme de trompette ;
▪ Une forme de ballon.

Un avantage est de disposer d'une forme permettant de bloquer le passage de la canule dans l'orifice de la paroi.

Selon un mode de réalisation, l'élément déformable prolonge l'orifice distal de la lumière d'injection, ledit élément déformable comprenant un embout distal fermé.

Selon un mode de réalisation, l'embout distal fermé réalise une fonction de support pour un dispositif de guidage introduit dans la lumière d'injection et exerçant une force d'appui sur ledit embout distal étant capable d'entrainer l'extension de l'élément déformable dans le sens longitudinal prolongeant l'axe de la lumière d'injection.

Selon un mode de réalisation, les lumières d'aspiration et la lumière d'injection sont coaxiales, la lumière d'aspiration ayant un diamètre supérieur au diamètre de la lumière d'injection. Un avantage de cette configuration est que le dispositif de canule peut être guidé simplement. Le guidage d'une lumière entrainant le guidage de la seconde lumière.

Selon un mode de réalisation, la lumière d'aspiration permet une circulation d'un fluide entre la surface externe de la lumière d'injection et la surface interne de la lumière d'aspiration sur au moins une portion du dispositif de canule.

Selon un mode de réalisation, la jonction entre la lumière d'aspiration et la lumière d'injection est réalisée par une diminution progressive du diamètre de la lumière d'aspiration sur au moins une portion de sorte à réaliser un second moyen de maintien maintenant le dispositif de canule dans une direction opposée à une force résultante du maintien du second agencement. Selon un mode de réalisation, les deux lumières sont collées sur une portion circonférentielle de la surface extérieure de la lumière d'injection. Selon un autre mode de réalisation, elles sont réalisées conjointement par exemple par moulure.

Selon un mode de réalisation, la jonction entre la lumière d'aspiration et la lumière d'injection forme une butée comprenant une augmentation du diamètre du dispositif de canule formant un troisième moyen de maintien maintenant le dispositif de canule dans une direction opposée à une force résultante du maintien du second agencement.

Selon un mode de réalisation, la lumière d'aspiration comprend une pluralité d'orifices agencés à sa périphérie.

Selon un mode de réalisation, le dispositif de canule comprend un dispositif de guidage coopérant avec l'embout de la lumière d'injection pour provoquer une extension de l'élément déformable dans le sens longitudinal prolongeant l'axe de la lumière d'injection.

Un autre objet de l'invention concerne un poumon artificiel comportant :
▪ un dispositif de canule de l'invention,
▪ une pompe agencée pour assurer le pompage d'un débit de fluide prédéterminé, ladite lumière d'aspiration coopérant avec une entrée de la pompe via un tube d'interface ;
▪ un oxygénateur agencé pour introduire une proportion d'oxygène dans un fluide, une entrée de l'oxygénateur coopérant avec une sortie de la pompe via un second tube d'interface ;
▪ un échangeur de chaleur agencé pour injecter l'oxygène fournit par l'oxygénateur à une température fixée dans un volume de fluide injectée dans une lumière d'injection du dispositif de canule par un troisième tube d'interface, ledit échangeur étant relié à l'oxygénateur et au dispositif de canule via un quatrième tube d'interface.

Selon un mode de réalisation, au moins un tube est recouvert d'une couche d'héparine.

Selon un mode de réalisation, la pompe est agencée et configurée pour établir un débit de sang pulsé.

Selon un mode de réalisation, l'oxygénateur comprend un revêtement en fibres.

Selon un mode de réalisation, l'oxygénateur comprend des moyens pour établir un flux laminaire d'oxygène.

### BREVE DESCRIPTION DES FIGURES

D'autres caractéristiques et avantages de l'invention ressortiront à la lecture de la description détaillée qui suit, en référence aux figures annexées, qui illustrent :
- La figure 1 : une représentation schématique du dispositif de canule double lumière selon un premier mode de réalisation de l'invention ;
- La figure 2 : une représentation schématique du dispositif de canule double lumière selon un second mode de réalisation de l'invention ;
- La figure 3 : une représentation schématique du dispositif de canule double lumière selon un troisième mode de réalisation de l'invention ;
- Les figures 4 à 6 : une représentation de l'introduction d'un dispositif de canule double lumière du troisième mode de réalisation de l'invention ;
- Les figures 7A à 7C : différents modes de réalisation d'un stent agencé à l'extrémité d'une lumière d'injection du dispositif de canule de l'invention ;

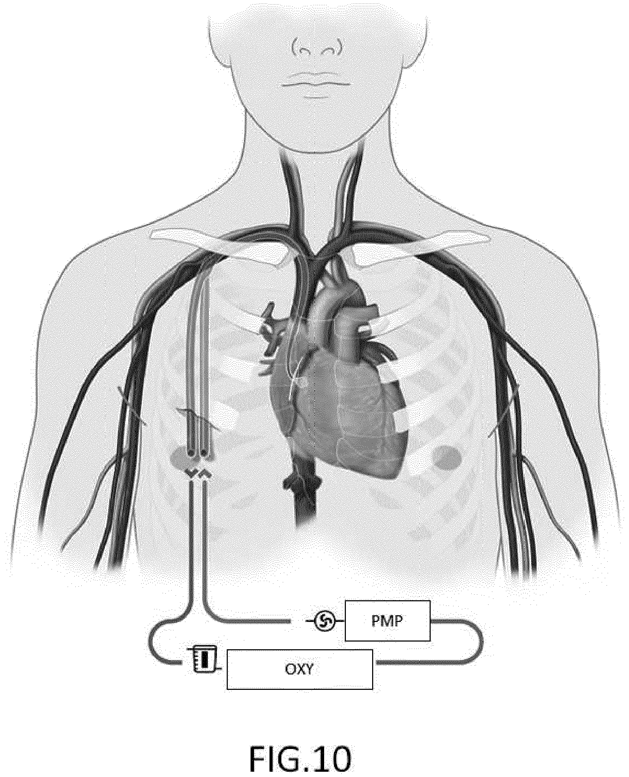

- Les figures 8 et 10: un système d'oxygénation par membrane extracorporelle (ECMO) relié à un coeur par l'intermédiaire du dispositif de canule selon un des modes de réalisation de l'invention ;

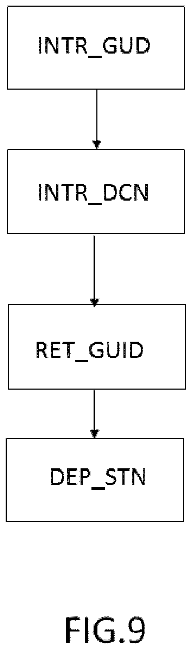

- La figure 9 : un digramme en bloc illustrant les étapes de mise en place du dispositif de canule de l'invention dans une région du coeur ;

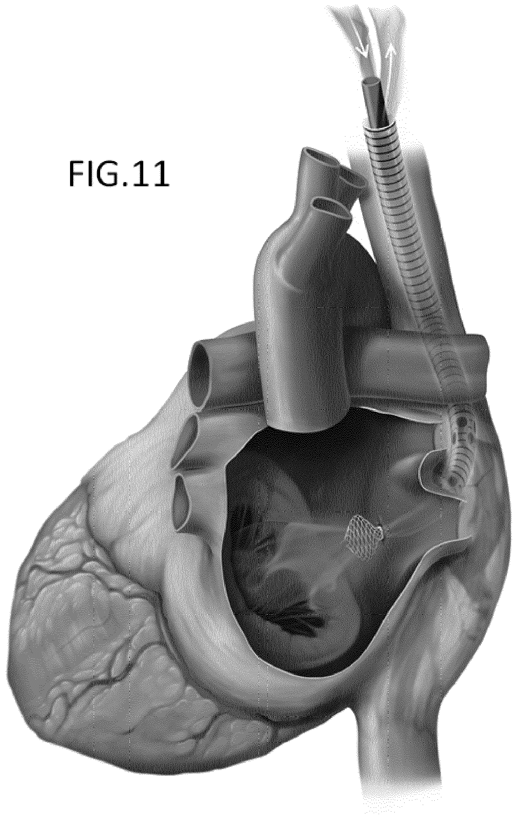

- La figure 11 : une représentation tridimensionnelle du dispositif de canule de l'invention lorsqu'il est introduit dans le coeur ;

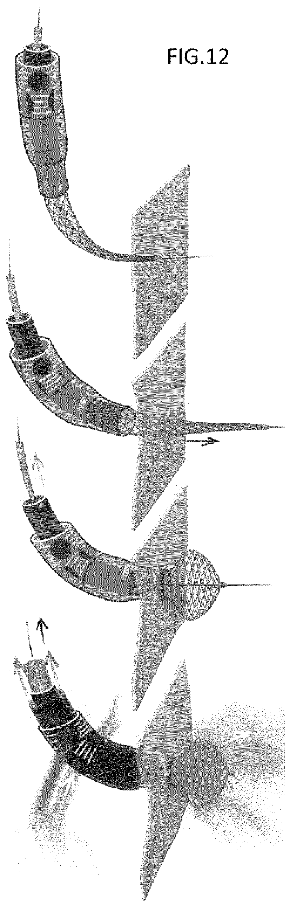

- La figure 12 : les différentes étapes d'introduction du dispositif de canule dans un orifice d'une paroi transseptale.

### DESCRIPTION DETAILLEE DE MODES DE REALISATION DE L'INVENTION

### Dispositif de canule

L'invention concerne un dispositif de canule double lumière LA et LI comprenant un élément déformable maillé qu'on désigne par « Stent » et noté STN, agencé à l'extrémité distale d'une des lumières. Une première lumière LI permet d'injecter du sang oxygéné dans un organe, elle est appelée lumière d'injection LI. La seconde lumière LA permet d'aspirer du sang pauvre en oxygène qui sera ré-oxygéné pour être réintroduit. Le Stent STN est maintenu à l'extrémité distale de la lumière d'injection LI. Il réalise notamment une fonction de maintien de la lumière LI dans l'organe ou l'artère qui est visé. Cette fonction est vitale compte tenu que la lumière LI doit être constamment maintenue dans un organe dans lequel le sang riche en oxygène est injecté.

Différents modes de réalisations sont décrits au regard des figures. Afin de mieux comprendre les figures, notons que le dispositif de canule 1 est destiné à faire circuler du sang entre un coeur et un poumon artificiel. Une première lumière LA est destinée à aspirer du sang vers le poumon artificiel, une seconde lumière LI est destinée à injecter du sang dans le coeur ou une artère.

### Lumières

On entend par « double lumière », la présence de deux éléments creux comme par exemple des tubes. Chaque lumière comprend au moins deux orifices dont un orifice à chaque extrémité. Les orifices peuvent être réalisés soit sur la circonférence d'une extrémité d'un tube fermé, soit à l'extrémité d'un tube ouvert. Les deux possibilités de réalisation d'orifices peuvent être également combinées. Les orifices permettent d'acheminer le sang soit vers un organe soit vers un composant du poumon artificiel. Le dispositif de canule 1 comprend deux lumières LI et LA réalisant donc chacune une fonction de canule. On parle indifféremment de « lumière » ou de « canule » dans la suite de la description.

Une lumière est un élément tel qu'une gaine comportant au moins deux orifices à chacune de ses extrémités. Elle peut être réalisée en une partie ou peut comprendre plusieurs parties cylindriques creuses se prolongeant. Si la lumière comprend différentes parties, chaque partie est solidaire des autres parties de sorte à former un élément monobloc creux E1 s'étendant.

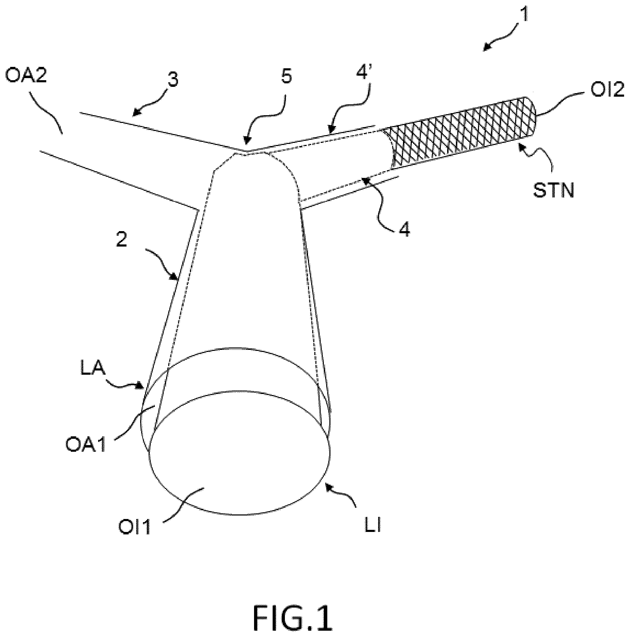

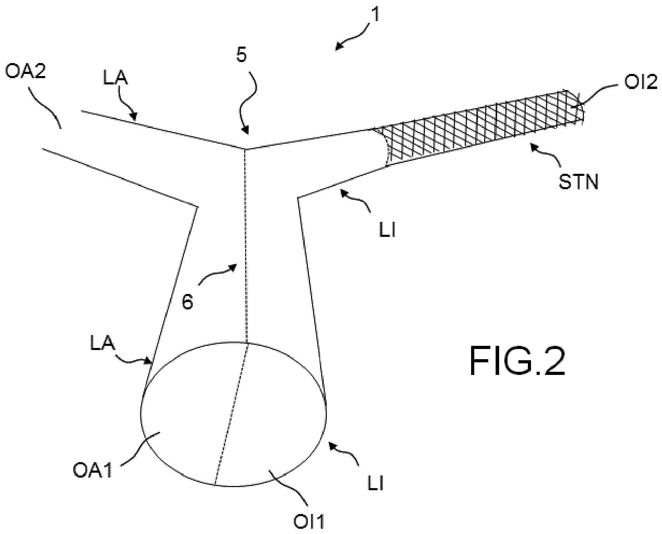

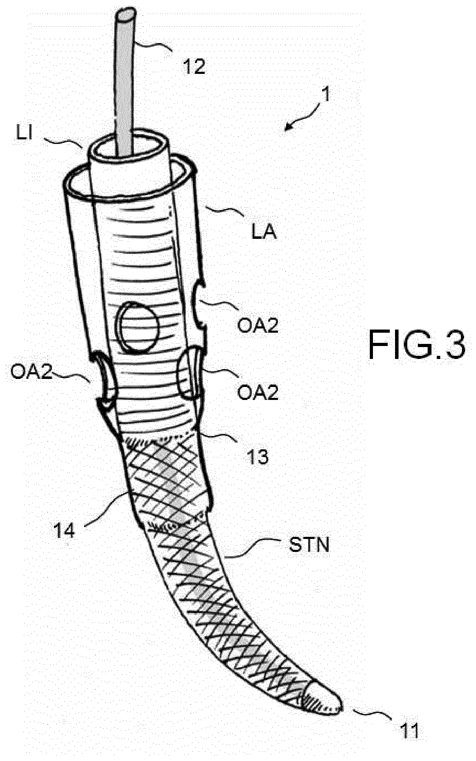

Selon un mode de réalisation, la forme d'un dispositif de canule 1 comportant deux lumières solidaires sur une portion dudit dispositif peut prendre la forme d'un « Y allongé » dans le cas de la figure 1 ou 2. Selon un autre mode de réalisation, les lumières peuvent être coaxiales et ne pas se séparer en forme de Y comme l'illustre le mode de réalisation de la figure 3.

Le dispositif de canule 1 comprend des lumières ayant une certaine souplesse et flexibilité pour permettre leur guidage dans le corps d'un patient. Le guidage peut être assuré par un ou plusieurs dispositif(s) de guidage qui est/sont introduit(s) dans au moins une lumière du dispositif de l'invention.

### Orifices

En outre, chaque lumière comporte des orifices :
- un orifice OI1 correspond à l'orifice de l'extrémité proximale de la lumière d'injection LI qui coopère avec un dispositif de poumon artificiel de type ECMO.
- un orifice OI2 correspond à l'orifice de l'extrémité distale de la lumière d'injection LI qui permet l'injection du sang oxygéné dans le coeur ou une artère.
- un orifice OA1 correspond à l'orifice de l'extrémité proximale de la lumière d'aspiration LA qui coopère avec un dispositif de poumon artificiel de type ECMO.
- un orifice OI2 correspond à l'orifice à l'extrémité distale de la lumière d'aspiration LA qui permet l'aspiration du sang pauvre en oxygène.

Chaque lumière peut comprendre à l'une de ses extrémités un ou plusieurs orifices.

Il existe différents dispositifs de canule double lumière. Les figures 1, 2 et 3 représentent trois modes de réalisation d'un dispositif de canule double lumières de l'invention. En ce qui concerne la coopération du Stent STN avec l'extrémité de la lumière d'injection LI, le mode de réalisation de la figure 3 peut se combiner aux modes de réalisation des figures 1 et 2.

### Dispositif de canule double lumière : 1^{er} mode

Le premier mode de réalisation représente un dispositif de canule 1 comprenant deux lumières LI et LA concentriques sur une première portion 2 et divergent sur une seconde portion 3 et 4 du dispositif de canule. Selon un mode de réalisation, le dispositif de canule 1 se sépare en deux lumières distincts 3 et 4 à partir de la portion commune 2. Chaque lumière est acheminée dans une région souhaitée d'une artère, d'une veine ou d'un organe pour y injecter ou prélever un volume sanguin.

Selon une variante de réalisation, le corps d'une première lumière, par exemple celle d'aspiration LA, peut se diviser en deux parties de sorte à se prolonger sur une partie de la seconde lumière comme cela est représentée par la partie 4' de la figure 1.

Dans ce mode de réalisation, la lumière d'injection LI est prolongée en son extrémité par un Stent STN qui a pour fonction le maintien de la lumière dans un organe ou une artère une fois cette dernière positionnée pour fonctionner et injecter du sang.

Le Stent STN est agencé à l'extrémité de la lumière d'injection LI lors de l'introduction du dispositif de canule 1. Il peut être, par exemple, mis sous tension ou compressé pour être déformé et faciliter ainsi l'introduction de la lumière d'injection LI dans la cavité souhaitée par l'intermédiaire d'un orifice. Une fois agencé, le dispositif de canule 1 est configuré de sorte à ce que le Stent STN maintienne la lumière d'injection LI dans une cavité telle qu'une oreillette ou une artère.

Le Stent STN peut être, par exemple, mis sous tension lors de l'introduction du dispositif de canule 1 et relâché lorsqu'il est positionné pour son fonctionnement.

### Dispositif de canule double lumière : 2^{ème} mode

Dans ce second mode de réalisation, les lumières d'injection LI et d'aspiration LA sont maintenues fixées l'une à l'autre sur une portion commune du dispositif de canule 1 de l'invention. Ce mode de réalisation permet de disposer de deux canaux propres pour chacune des lumières LI et LA et de configurer un débit indépendant pour l'une et l'autre des lumières.

Une séparation des lumières 5 permet d'acheminer la lumière d'injection LI dans une cavité pour l'injection d'un volume de sang oxygéné et d'acheminer la lumière d'aspiration LA pour le prélèvement d'un volume de sang pauvre en oxygène.

Les orifices OI1, 012 et OA1 et OA2 sont représentés sur la figure 2 de manière similaire à la figure 1.

Aux extrémités distales des lumières LI et LA, la lumière peut comprendre un ou plusieurs orifice(s) selon les variantes de réalisation. Les orifices peuvent être agencés sur l'extrémité longitudinale distale de la lumière ou encore agencés latéralement sur la circonférence d'une lumière.

A titre d'exemple, le Stent représenté aux figures 1 et 2 peut recouvrir une pluralité d'orifices, l'extrémité longitudinale de la lumière LI étant fermée.

### Dispositif de canule double lumière : 3^{ème} mode

La figure 3 représente une mode de réalisation particulièrement avantageux. Les lumières LI et LA sont concentriques sur toute la longueur de la lumière d'aspiration LA. La lumière d'aspiration comprend une terminaison 13 permettant de se confondre avec la surface extérieure de la lumière d'injection. Cette terminaison peut être obtenue par fabrication et assemblage des gaines formant les lumières assurant une étanchéité entre les deux lumières LI et LA.

Dans ce mode de réalisation, la lumière d'injection prolonge dans la continuité de l'axe commun aux deux lumières concentriques une portion 14 recouverte par un Stent STN. Le STN est avantageusement fixé à la partie distale de la lumière d'injection par couture, par collage ou encore par n'importe quel procédé permettant de fixer un Stent STN à une gaine pour canule.

Le dispositif de canule 1 dans ce mode de réalisation comprend à son extrémité distale un Stent STN qui est fermé à son extrémité par un embout 11. Le Stent STN comprend des orifices pour l'injection d'un sang riche en oxygène. Notamment, lorsque le Stent STN forme un tissu maillée extensible, les orifices sont les mailles du Stent STN.

Dans ce mode de réalisation, la lumière d'aspiration LA comprend une pluralité d'ouvertures OA2 qui permet de prélever un volume de sang pauvre en oxygène.

Dans ce mode de réalisation, on comprend que l'extrémité 11 permet de former un point d'appui au dispositif de guidage 12 qui est introduit dans le dispositif de canule 1 de l'invention pour l'acheminer dans la cavité dans laquelle elle fonctionnera avec le poumon artificielle.

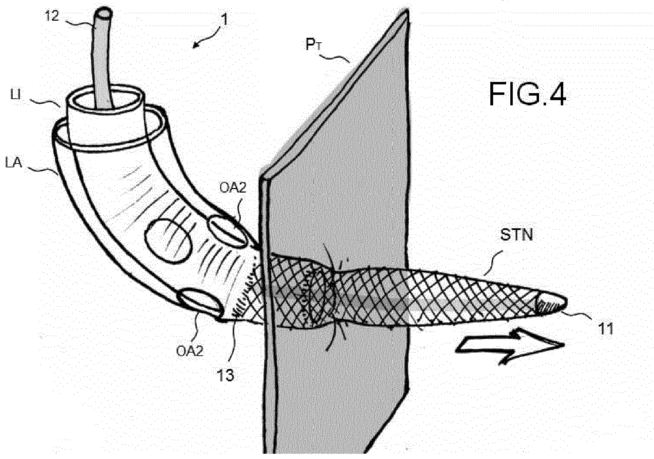

La figure 4 illustre l'introduction du dispositif de canule 1 dans une paroi telle qu'une paroi séparant les oreillettes du coeur pour l'injection d'un volume de sang oxygéné. Selon un mode de réalisation, la lumière d'injection LI est introduite dans l'oreillette gauche OG du coeur par la paroi transseptale P_{T} qui est représentée à la figure 4 par l'annotation P_{T}.

Le perçage de la paroi transseptale peut être réalisé préalablement à l'introduction du dispositif de canule de l'invention.

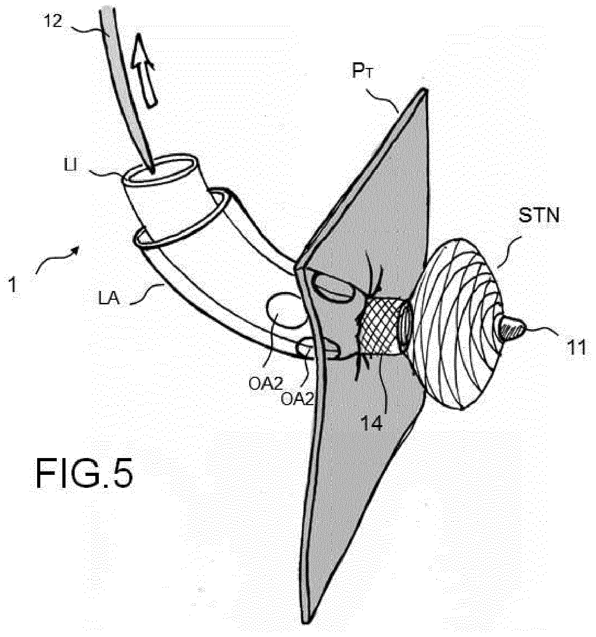

La figure 5 illustre après le positionnement de la lumière d'injection LI, le retrait du dispositif de guidage 12 et le déploiement du Stent STN dans une position de maintien. Dans ce mode de réalisation, le Stent prend le volume, par exemple, d'un ovoïde ou d'un champignon permettant à la lumière d'injection LI de se maintenir dans l'oreillette gauche OG. Un avantage d'un tel dispositif de canule 1 est que ce dispositif permet de surmonter les mouvements induits par les battements du coeur et de l'oreille gauche qui se contracte et se relâche régulièrement et causant une force tendant à repousser la lumière d'injection LI vers l'orifice de la paroi transseptale P_{T}.

Sur la figure 5, la portion 14 de la lumière d'injection LI est recouverte du Stent STN. Sur cette portion la lumière d'injection LI et le Stent STN sont solidaires et rendus fixes grâce à une colle ou une couture par exemple. Le Stent STN se prolonge sur une portion qui a changé de forme du fait du retrait du dispositif de guidage 12. La forme du Stent qui se prolonge au-delà de la lumière d'injection a un volume plus important que le diamètre de l'orifice de la paroi P_{T} une fois son déploiement réalisé.

Selon un mode de réalisation, le stent STN est introduit en tension grâce au dispositif de guidage 12 qui permet d'étendre le Stent sur toute sa longueur lors de son introduction dans l'orifice de la paroi P_{T}. Lorsque le dispositif de guidage 12 est retiré, le Stent qui a une mémoire de forme reprend un volume initial, en forme d'ovoïde ou de champignon dans l'exemple de la figure 5.

D'autres formes de Stent sont envisageables à partir du moment où le diamètre de la forme du Stent en position déployée est supérieur au diamètre de l'orifice formé dans la paroi transseptale.

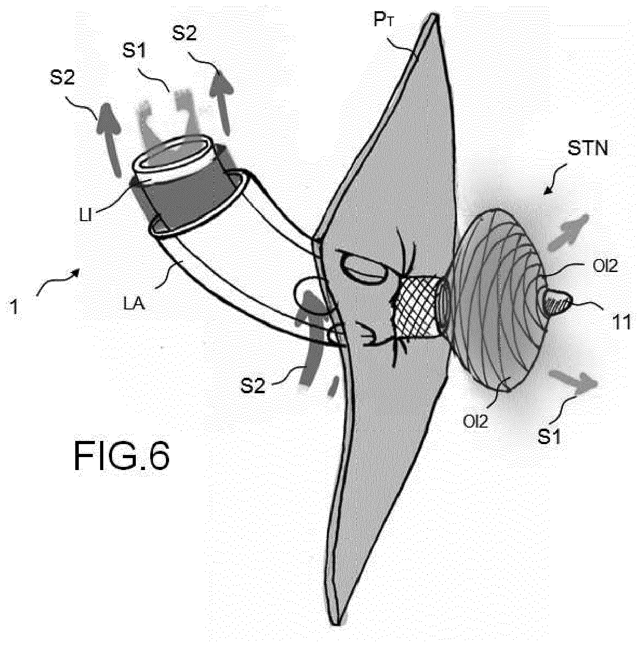

La figure 6 représente un mode de fonctionnement lorsque le dispositif de canule est agencé convenablement, c'est-à-dire avec la lumière d'injection LI dans l'oreillette gauche OG. La figure 6 permet de représenter le volume de sang oxygéné S1 qui est introduit dans l'oreillette gauche OG, c'est-à-dire au-delà de la paroi transseptale PT notamment par l'orifice distal OI2 de la lumière d'injection LI et des orifices du Stent STN formant des mailles ouvertes en position de déploiement.

Le flux S2 aspiré par les orifices OA2 et remontant via la lumière LA jusqu'au poumon artificiel est représenté.

### Stent STN

On entend par « Stent », un élément déformable préférentiellement par extension et maillé. Un tel élément est comparable à un ressort, pouvant se déformer puis reprendre sa forme de repos.

Dans un mode réalisation, le Stent STN est à mémoire de forme. On entend par mémoire de forme un élément pouvant être compressé ou étiré lorsqu'une pression/tension lui est appliquée. Lors de son relâchement, le stent STN peut prendre une forme donnée/fixée telle qu'une trompette, un champignon etc.

Un avantage est lorsque l'on souhaite retirer le dispositif de canule, un dispositif de guidage 12 peut être réintroduit dans la lumière d'injection LI. Lorsqu'il est réintroduit, il peut exercer sur l'embout distal 11 permet de former une surface d'appui, le dispositif de guidage permettant l'élongation du Stent STN. Le Stent ainsi allongé peut être retiré via l'orifice de la paroi transseptale P_{T}.

Ainsi, lors de son introduction dans l'oreillette gauche OG par perçage d'un trou dans la paroi transseptale auriculaire, le Stent STN est :
- soit maintenu étendu par extension du tissu maillée comme cela est représenté dans les exemples des figures 5 et 6 ;
- soit maintenu comprimé ou compressé, de sorte à épouser la forme du dispositif de canule 1. Une telle compression permet l'introduire du dispositif de canule 1 solidaire du stent STN à travers le trou réalisé dans la paroi.

Dans le dernier cas de figure, le Stent récupère sa forme initiale, par exemple par mémoire de forme, dont le diamètre est devient alors plus grand que l'orifice de la paroi percée.

Selon un mode de réalisation, le Stent STN est métallique, par exemple en acier inoxydable ou encore réalisé avec un alliage cobalt-chrome ou platine-chrome ou encore de nitinol.

Selon un autre mode de réalisation, le Stent STN est fabriqué dans un matériau élastomère ou encore un matériau composite.

Selon un mode de réalisation, le Stent STN présente une surface maillée.

Selon le mode de réalisation, le Stent STN est maintenu à l'extrémité distale de la lumière d'injection LI sur une portion de sa surface extérieure comme la portion 14 des figures 3 et 5. Selon une variante de réalisation, le Stent STN peut être maintenu sur une portion de la surface intérieure de la lumière d'injection LI.

Dans ces modes de réalisation, le Stent STN prolonge la lumière d'injection LI au-delà de l'extrémité distale de ladite lumière LI. Dans ce cas, le Stent STN ne se superpose pas sur la dernière portion à la lumière d'injection LI.

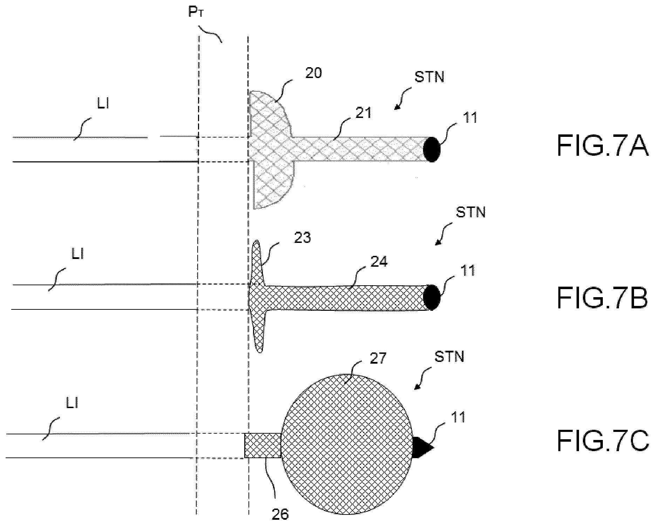

La figure 7A représente un mode de réalisation d'un Stent STN de l'invention. Il comprend une partie 20 à mémoire de forme ayant une forme de champignon et permettant de retenir la lumière d'injection LI dans une cavité une fois cette dernière positionnée. Le Stent STN comprend une partie 21 qui prolonge la partie 20. Sur ce prolongement, la lumière LI peut être prolongée également. Un embout distal 22 permet de fermer la lumière LI et de servir d'élément de guidage lorsque le dispositif de guidage 12 du dispositif de canule 1 est actionné pour le positionnement de la lumière LI. Lorsque la lumière LI prolonge également la portion comprenant le Stent STN, cette dernière peut comprendre avantageusement des orifices permettant le passage du sang oxygéné au travers de ces derniers puis au travers des mailles du Stent STN.

Selon le mode de réalisation, le diamètre du Stent STN augmente sur une portion du Stent STN seulement selon la mémoire de forme réalisée. Une telle forme permet au Stent STN et ainsi à la lumière d'injection LI de rester sensiblement fixe dans l'oreillette gauche OG. En effet, le diamètre du stent STN étant supérieur à celui du trou réalisé dans la paroi transseptale, le stent STN reste collé contre la paroi séparant le septum de l'oreillette gauche OG, empêchant le dispositif de canule1 de bouger et de se retirer par l'orifice de la paroi transseptale.

La figure 7B représente une variante de réalisation de la figure 7A dans laquelle la forme du Stent à mémoire de forme prend la forme d'une assiette ou de « trompette » dont le diamètre est supérieur au diamètre de l'orifice de la paroi transseptale P_{T}.

De la même manière que pour le mode de réalisation représenté à la figure 7A, cette forme de Stent STN sur une portion dudit Stent STN, permet de fixer le dispositif de canule 1 dans une cavité du coeur en empêchant celle-ci de se déplacer malgré les mouvements de contraction et de relâchement du coeur.

La figure 7C représente un autre mode de réalisation sensiblement proche des modes des figures 5 et 6. Dans ce cas de figure, la lumière d'injection peut se prolonger sur la portion 26. La portion 27 non fixée à la lumière LI, soit parce qu'elle ne se prolonge pas dans cette portion, soit parce qu'elle n'est pas fixée à la lumière qui se prolonge, prend une forme de ballon lorsque le dispositif de guidage 12 est retiré.

Le Stent STN doit être suffisamment solidarisé du dispositif de canule 1 de sorte que lorsque le coeur d'un patient est en mouvement, le Stent STN ne risque pas de rester dans l'oreillette gauche OG alors que le dispositif de canule 1 est éjectée de l'oreillette gauche par l'intermédiaire de l'orifice de la paroi transseptale. Ainsi, la fixation du Stent STN sur la lumière d'injection est réalisée de sorte à résister à une force de plusieurs Newton.

### Matériau

Selon un mode de réalisation, le matériau utilisé pour la fabrication des lumières du dispositif de canule est en PVC semi-rigide thermosensible.

### Système ECMO

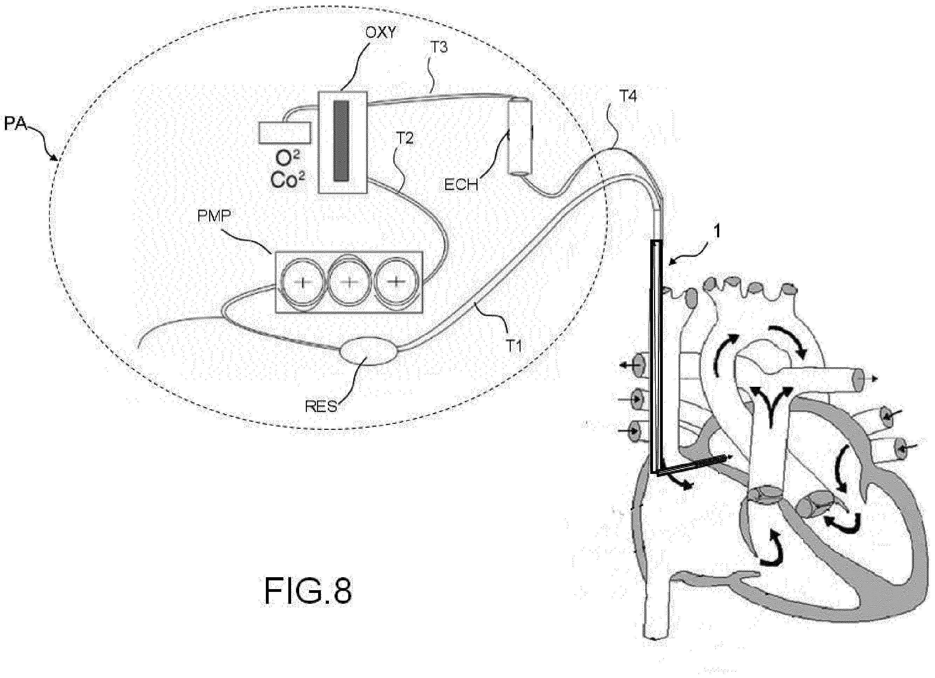

En référence à la figure 8, le système ECMO comprend :
- un dispositif de canule 1,
- une pompe PMP,
- un oxygénateur OXY,
- un échangeur de chaleur ECH,
- quatre tubes : T1, T2, T3 et T4.
- **Mise en place du dispositif de canule, fonctionnement** (non revendiqué et à titre d'illustration)

Le dispositif de canule 1 est introduit dans une région du coeur par l'intermédiaire de la veine cave supérieure. Selon un autre mode de réalisation, le dispositif de canule 1 est introduit dans une région du coeur par l'intermédiaire de la veine cave inférieure

Selon le mode de réalisation, le dispositif de canule 1 comprend deux lumières LI et LA sont indépendantes tout en étant solidarisées l'une à l'autre sur une portion du dispositif de canule 1 de sorte qu'elles puissent recevoir, en parallèle, des fluides différents se déplaçant à des débits différents.

Ainsi, la lumière d'aspiration du dispositif de canule 1 prélève un volume de sang pauvre en oxygène et riche en dioxyde de carbone à un débit fixé. Le sang pauvre en oxygène provient de l'oreillette droite OD du coeur dans laquelle débouche l'orifice OA2 de la lumière d'aspiration LA. Le sang provenant de l'oreillette droite OD est ensuite transporté vers un système d'oxygénation par membrane extracorporelle ECMO par l'orifice OA1 de la lumière d'aspiration LA. En effet, théoriquement, l'oreillette droite OD collecte le sang qui a parcouru tout le corps et l'envoie vers le ventricule droit VD afin qu'il soit éjecté dans les poumons pour y être oxygéné. Selon l'invention, avant que le sang pauvre en oxygène contenu dans l'oreillette droite OD ne soit envoyé vers le ventricule droit, le sang est pompé pour subir une oxygénation par le système ECMO.

La lumière d'injection LI du dispositif de canule 1 reçoit du sang riche en oxygène à un débit fixé. L'enrichissement du sang en oxygène résulte d'un traitement par le système ECMO qui réalise une oxygénation du sang provenant de la lumière d'aspiration LA. Le sang riche en oxygène est introduit dans la lumière d'injection LI du dispositif de canule 1 par l'intermédiaire de l'orifice LI1 puis passe le long de la lumière LI jusqu'à l'orifice LI2 débouchant dans la cavité de l'oreillette gauche OG. En effet, théoriquement, l'oreillette gauche OG a pour rôle de collecter le sang qui a traversé les poumons pour l'oxygéner puis de l'acheminer au ventricule gauche VG qui éjecte le sang oxygéné dans l'ensemble du corps.

### - Réservoir

Selon un mode de réalisation, le système ECMO comprend un réservoir veineux RES. Ce dernier a pour fonction de réguler le début de sang traité par le poumon artificiel. La lumière d'aspiration du dispositif de canule 1 prélève un volume de sang qui est acheminé jusqu'au réservoir veineux. Afin de réaliser un drainage du sang provenant du de l'oreillette droite OD du coeur. Selon un mode de réalisation, cette étape est réalisée avant l'étape de pompage explicité ci-après.

### - Pompe PMP

Ladite pompe PMP assure deux rôles : le pompage du sang pauvre en oxygène et riche en dioxyde de carbone provenant de la lumière d'aspiration LA du dispositif de canule 1 ainsi que l'injection du sang oxygéné dans la lumière d'injection LI du dispositif de canule 1.

La pompe PMP est configurée pour pomper le sang pauvre en oxygène à un débit propre au besoin de pompage du coeur. Le débit doit permettre une décarboxylation efficace réalisée à partir du dioxyde de carbone contenu dans le sang pauvre en oxygène.

La pompe PMP est également configurée pour que le sang injecté dans la lumière d'injection LI soit à un débit physiologique c'est-à-dire semblable à celui d'un poumon « naturel ».

Selon un exemple de réalisation, la pompe PMP est de type « centrifuge ». Une pompe PMP centrifuge utilise le mouvement de rotation d'une roue insérée dans la pompe PMP. La roue, en tournant à une vitesse élevée, déplace le fluide par effet centrifuge et augmente sa pression. Ainsi, le déplacement ainsi que le débit du fluide peuvent être fixés et contrôlés.

Selon un mode de réalisation, la pompe PMP est « à galets » également appelée « péristaltique ». Une pompe PMP à galets est constituée d'une tête soutenant un tuyau flexible contenant le fluide ainsi qu'un rotor comprenant des galets. Les galets déforment et obturent le tuyau pendant la rotation ce qui a pour conséquence d'entraîner le déplacement du fluide se trouvant entre deux galets. Ainsi, le déplacement et le débit du fluide peuvent être fixés et contrôlés.

Selon un mode de réalisation, la pompe PMP génère un débit pulsé. En effet, le débit pulsé est plus physiologique, il permet de préserver les cellules humaines lors de leur passage dans l'oxygénateur OXY.

Selon un mode de réalisation, la pompe PMP est miniaturisée de sorte à minimiser l'encombrement du système ECMO et ainsi permettre à un patient de l'utiliser pendant une plus longue durée.

Selon un mode de réalisation, la pompe PMP est biocompatible de sorte qu'un poumon artificiel puisse être introduit et accepté par un corps humain.

### Oxygénateur OXY

L'oxygénateur OXY comporte une membrane qui reproduit artificiellement la fonction de la membrane alvéolo-capillaire. Cette membrane permet de réaliser des échanges gazeux afin d'oxygéner le sang et d'éliminer le dioxyde de carbone contenu dans le sang par décarboxylation. L'oxygénateur OXY est relié à la pompe PMP à partir de laquelle il reçoit le sang pauvre en oxygène et riche en dioxyde de carbone à un débit fixé.

Selon un mode de réalisation, la membrane de l'oxygénateur OXY est plane. Les oxygénateurs OX à membranes planes comportent des membranes en silicone ou des membranes assemblées en couches.

Selon un mode de réalisation, la membrane de l'oxygénateur OXY est tubulaire. Ces types d'oxygénateurs OX sont composés de fibres creuses composées, par exemple de polyméthylpentènes non poreuses.

Selon un mode de réalisation, les fibres peuvent comprendre un revêtement offrant moins de résistance à l'écoulement et favorisant un écoulement d'un flux laminaire.

Selon un mode de réalisation, les cellules endothéliales sont semées dans l'oxygénateur pour offrir un bio-oxygénateur. Le flux pulsatile de la pompe favorise la croissance des cellules endothéliales.

Selon un mode de réalisation, des micro-fluides peuvent reproduire les vaisseaux capillaires des poumons pour faciliter l'endothélialisation.

Selon un mode de réalisation, l'oxygénateur OXY est miniaturisé de sorte à minimiser l'encombrement du système ECMO et ainsi permettre à un patient de l'utiliser pendant une plus longue durée.

### Echangeur de chaleur ECH

L'échangeur de chaleur ECH reçoit le sang oxygéné de l'oxygénateur OXY pour le réchauffer. En effet, le sang passe par un système permettant de transférer l'énergie thermique d'un fluide tel que l'eau vers le sang, sans mélanger les deux fluides. Le flux thermique traverse la surface d'échange qui sépare le sang oxygéné et l'eau. L'échangeur thermique ECH fixe la température du sang afin qu'elle soit comprise dans l'intervalle de température du sang circulant dans le corps d'un patient.

Selon un mode de réalisation l'échangeur de chaleur ECH est relié à un réservoir veineux de sorte à réchauffer le sang après son drainage, les étapes de pompage puis d'oxygénation sont alors réalisées par la suite.

L'orifice OI1 sur l'extrémité proximale de la lumière d'injection LI du dispositif de canule 1 est relié à l'échangeur thermique ECH de sorte qu'il reçoive le sang oxygéné chauffé. Le sang oxygéné et chauffé passe le long de la lumière d'injection LI jusqu'à l'orifice OI2 de son extrémité distale, longitudinale et/ou circonférentielle.

De manière alternative, selon un autre mode de réalisation, le chauffage peut provenir de l'oxygénateur lui-même. Dans ce cas, il comprend avantageusement des résistances prévues à cet effet.

### Tubes T1, T2, T3, T4

La pompe PMP, l'oxygénateur OXY et l'échangeur thermique ECH et le dispositif de canule 1 sont reliés entre eux par l'intermédiaire de quatre tubes T1, T2, T3 et T4. Ces tubes T1, T2, T3, T4 sont destinés à faire passer le sang oxygéné ou non entre les différents éléments permettant son traitement.

Ainsi, le tube T1 permet de relier l'orifice OA1 de la lumière d'aspiration du dispositif de canule 1 à la pompe PMP. Le tube T2 permet de relier la pompe PMP à l'oxygénateur OXY. Le tube T3 permet de relier l'oxygénateur OXY à l'échangeur thermique ECH et le tube T4 permet de relier l'échangeur thermique ECH à l'orifice OA1 de la lumière d'injection du dispositif de canule 1.

Selon un mode de réalisation, les tubes T1, T2, T3, T4 sont dimensionnés pour être les plus courts possibles de sorte à minimiser l'encombrement. Ainsi, la longueur des tubes T1, T2, T3, T4 est préférentiellement réduite à une longueur minimale pour diminuer l'encombrement du dispositif. De plus, le diamètre desdits tubes T1, T2, T3, T4 est choisi de sorte à permettre au sang de se déplacer tout en minimisant les risques d'hémolyse et de coagulation. Ainsi, le diamètre des tubes T1, T2, T3, T4 est ajusté selon les débits connus permettant la circulation du sang dans le corps.

Selon un mode de réalisation, les tubes T1, T2, T3, T4 ont un revêtement en héparine, un anticoagulant qui permet ainsi d'éviter la formation de caillots de sang à l'intérieur des tubes T1, T2, T3, T4.

### Procédé (non revendiqué et à titre d'illustration)

La figure 4 représente les étapes de mise en place du dispositif de canule 1 dans une cavité du coeur, l'oreillette gauche.

Selon une première étape, INTR_GUD, un guide de perçage est introduit dans la veine cave supérieure, ledit guide de perçage étant surmonté d'une aiguille. Ledit guide de perçage passe le long de la veine cave supérieure jusqu'à atteindre l'oreillette droite OD. L'aiguille du guide de perçage est alors orientée vers la paroi transseptale auriculaire, du côté de l'oreillette droite, afin d'y réaliser un orifice. L'orifice permet de faire communiquer l'oreillette droite OD et l'oreillette gauche OG pour préparer l'introduction de la lumière d'injection Ll.

Le perçage de l'orifice est réalisé en utilisant une manoeuvre de Rachkind.

Le perçage de l'orifice est contrôlé par radiographie ou tout autre système d'imagerie tel qu'un dispositif d'ultrason tel qu'une échographie ou par un scanner.

Les dimensions de l'aiguille sont choisies de sorte à minimiser le diamètre de l'orifice réalisé dans la paroi tout en permettant l'introduction du dispositif de canule 1, notamment de la lumière d'injection dans l'orifice.

Le procédé de l'invention peut être réalisé à partir de l'étape INT_DCN, l'étape de perçage pouvant être réalisé préalablement ou conjointement avec l'introduction du dispositif de canule 1. En d'autres termes, l'étape de perçage est une étape optionnelle du procédé de l'invention.

Selon une seconde étape, INTR_DCN, le dispositif de canule 1 est introduit dans la veine cave supérieure jusqu'à l'oreillette gauche OG au moyen d'un dispositif de guidage 12. Cette étape est illustrée à la figure 4. Le dispositif de guidage 12 est introduit à l'intérieur de la lumière d'injection LI dudit dispositif de canule 1 et prend appui sur un embout distal 11 pour entrainer ladite lumière LI. Le dispositif de guidage 12 maintien, dans cet exemple, le Stent STN en position étendue, son diamètre étant alors maintenu le plus faible possible pour qu'il puisse pénétrer dans l'orifice de la paroi transseptale. La lumière d'injection LI est introduite jusqu'à une butée 13 qui forme la jonction circonférentielle de la lumière d'aspiration LA avec la lumière d'injection LI. Avantageusement, la butée est formée par une augmentation du diamètre progressive sur une portion du dispositif de canule 1 permettant de maintenir en place dans l'orifice ledit dispositif de canule 1. Dans une variante de réalisation, l'augmentation du diamètre à la jonction circonférentielle 13 peut être abrupte de sorte à former un butée empêchant la lumière d'aspiration LA de pénétrer dans l'orifice de la paroi transseptale. Un avantage est de former une force de maintien dans le sens opposé à la force résultante du maintien provoqué par le Stent STN en position déployée conformément à l'étape de déploiement DEP_STN décrite ci-après.

Une fois la lumière d'injection positionnée comme cela est représenté à la figure 5, une étape de retrait du guide peut être entamée.

Selon une troisième étape, RET_GUID, le dispositif de guidage 12 est retiré. Le Stent STN n'étant plus maintenu sur l'extrémité distale en position d'extension, il reprend sa forme initiale par mémoire de forme. Le Stent STN prend une forme dont le diamètre est plus important que le diamètre de l'orifice. Cette étape de déploiement du Stent STN est notée DEP_STN. Cette étape résulte dans ce mode de réalisation du retrait du dispositif de guidage 12. Elle est donc réalisée en même temps que l'étape de retrait RET_GUID. Selon un mode de réalisation, le Stent STN met quelques secondes à reprendre sa mémoire de forme, selon un autre exemple, la Stent STN se déploie sans délai suite au retrait du dispositif de guidage 12.

Ainsi le dispositif de canule 1 est maintenu fixe d'une part par la butée 13 et d'autre part par le Stent STN faisant office d'un moyen de blocage.

Selon un autre mode de réalisation, le déploiement du Stent STN est réalisé par un autre dispositif introduit dans la lumière d'injection.

Lorsque la lumière d'injection est positionnée et maintenue dans l'oreillette gauche, la lumière d'aspiration peut prélever un volume sanguin dans l'oreillette droite grâce à au moins un orifice OA2 à sa circonférence.

## Revendications

1. Dispositif de canule (1) pour la circulation d'un fluide dans un poumon artificiel **caractérisé en ce qu'**il comprend :
- une première lumière (LA), dite lumière d'aspiration, comprenant au moins un orifice permettant l'aspiration d'un volume du fluide ;
- une seconde lumière (Ll), dite lumière d'injection, comprenant au moins un orifice permettant l'injection d'un volume du fluide,
**caractérisée en ce que** la lumière d'injection (LI) et la lumière d'aspiration (LA) sont maintenues solidaire sur au moins une portion de leur longueur, lesdites lumières étant étanches l'une de l'autre, la lumière d'injection (LI) comporte à son extrémité distale un élément déformable (STN) maillé de sorte à permettre le passage d'un fluide entre ses mailles, ledit élément déformable étant maintenu sur une portion de son extrémité distale, ledit élément déformable (STN) comportant un premier agencement dit de « passage » et un second agencement dit « de maintien », le passage du premier agencement au second agencement s'effectuant par une modification du diamètre dudit élément déformable (STN).

2. Dispositif de canule (1) selon la revendication 1, **caractérisé en ce que** l'élément déformable (STN) est maillé extensible et rétractable.

3. Dispositif de canule (1) selon l'une quelconque des revendications 1 à 2, **caractérisé en ce que** :
▪ le premier agencement de l'élément déformable (STN) est obtenu par extension dudit élément déformable (STN), le diamètre de l'élément déformable étant alors inférieur à un premier seuil ;
▪ le second agencement de l'élément déformable (STN) est obtenu dans sa forme de repos, le diamètre de l'élément déformable étant alors supérieur à un second seuil.

4. Dispositif de canule (1) selon l'une quelconques des revendications 1 à 3, **caractérisé en ce que** l'élément déformable (STN) est « à mémoire de forme ».

5. Dispositif de canule (1) selon la revendication 1, **caractérisé en ce que** l'élément déformable est un Stent (STN).

6. Dispositif de canule (1) selon la revendication 1, **caractérisé en ce que** l'élément déformable (STN) comprend une forme de repos réalisant un premier moyen de maintien du dispositif de canule (1), l'une des formes de repos pouvant être l'une des suivantes:
▪ Une forme de champignon ;
▪ Une forme d'assiette ;
▪ Une forme de trompette ;
▪ Une forme de ballon.

7. Dispositif de canule selon la revendication 1, **caractérisé en ce que** l'élément déformable (STN) prolonge l'orifice distal de la lumière d'injection (LI), ledit élément déformable (STN) comprenant un embout distal fermé (11).

8. Dispositif de canule selon la revendication 1, **caractérisé en ce que** l'embout distal fermé (11) réalise une fonction de support pour un dispositif de guidage (12) introduit dans la lumière d'injection (LI) et exerçant une force d'appui sur ledit embout distal (11) étant capable d'entrainer l'extension de l'élément déformable (STN) dans le sens longitudinal prolongeant l'axe de la lumière d'injection (LI).

9. Dispositif de canule selon la revendication 1, **caractérisé en ce que** la lumière d'aspiration (LA) et la lumière d'injection (LI) sont coaxiales, la lumière d'aspiration (LA) ayant un diamètre supérieur au diamètre de la lumière d'injection (LI).

10. Dispositif de canule selon la revendication 9, **caractérisé en ce que** la lumière d'aspiration (LA) permet une circulation d'un fluide entre la surface externe de la lumière d'injection (LI) et la surface interne de la lumière d'aspiration (LA) sur au moins une portion du dispositif de canule (1).

11. Dispositif de canule selon la revendication 10, **caractérisé en ce que** la jonction entre la lumière d'aspiration (LA) et la lumière d'injection (LI) est réalisée par une diminution progressive du diamètre de la lumière d'aspiration (LA) sur au moins une portion de sorte à réaliser un second moyen de maintien maintenant le dispositif de canule (1) dans une direction opposée à une force résultante du maintien du second agencement.

12. Dispositif de canule selon la revendication 10, **caractérisé en ce que** la jonction (13) entre la lumière d'aspiration (LA) et la lumière d'injection forme une butée comprenant une augmentation du diamètre du dispositif de canule (1) formant un troisième moyen de maintien maintenant le dispositif de canule (1) dans une direction opposée à une force résultante du maintien du second agencement.

13. Dispositif de canule selon la revendication 1, **caractérisé en ce que** la lumière d'aspiration (LA) comprend une pluralité d'orifices agencés à sa périphérie.

14. Dispositif de canule selon la revendication 1, **caractérisé en ce qu'**il comprend un dispositif de guidage (12) coopérant avec l'embout (11) de la lumière d'injection (LI) pour provoquer une extension de l'élément déformable dans le sens longitudinal prolongeant l'axe de la lumière d'injection (LI).

15. Poumon artificiel **caractérisé en ce qu'**il comporte :
▪ un dispositif de canule (1) selon l'une quelconque des revendications 1 à 14,
▪ une pompe agencée pour assurer le pompage d'un débit de fluide prédéterminé, ladite lumière d'aspiration (LA) coopérant avec une entrée de la pompe via un tube d'interface (T1) ;
▪ un oxygénateur agencé pour introduire une proportion d'oxygène dans un fluide, une entrée de l'oxygénateur coopérant avec une sortie de la pompe via un second tube d'interface (T2) ;
▪ un échangeur de chaleur agencé pour injecter l'oxygène fournit par l'oxygénateur à une température fixée dans un volume de fluide injectée dans une lumière d'injection (LI) du dispositif de canule (1) par un troisième tube d'interface (T3), ledit échangeur étant relié à l'oxygénateur et au dispositif de canule (1) via un quatrième tube d'interface (T4).

16. Poumon artificiel selon la revendication 15, **caractérisé en ce qu'**au moins un tube est recouvert d'une couche d'héparine.

17. Poumon artificiel selon l'une quelconque des revendications 15 à 16, **caractérisé en ce que** la pompe est agencée et configurée pour établir un débit de sang pulsé.

18. Poumon artificiel selon l'une quelconque des revendications 15 à 17, **caractérisé en ce que** l'oxygénateur comprend un revêtement en fibres.

19. Poumon artificiel selon l'une quelconque des revendications 15 à 18, **caractérisé en ce que** l'oxygénateur comprend des moyens pour établir un flux laminaire d'oxygène.

## Patentansprüche

1. Kanülenvorrichtung (1) zur Zirkulation eines Fluids in einer künstlichen Lunge, **dadurch gekennzeichnet, dass** sie Folgendes umfasst:
- ein erstes Lumen (LA), das sogenannte Ansauglumen, mit mindestens einer Öffnung, die das Ansaugen eines Volumens des Fluids gestattet;
- ein zweites Lumen (LI), das sogenannte Injektionslumen, mit mindestens einer Öffnung, die das Injizieren eines Volumens des Fluids gestattet,
**dadurch gekennzeichnet, dass** das Injektionslumen (LI) und das Ansauglumen (LA) über mindestens einen Teil ihrer Länge fest miteinander verbunden sind, wobei die Lumen gegeneinander abgedichtet sind, das Injektionslumen (LI) an seinem distalen Ende ein verformbares Element (STN) aufweist, das so vermascht ist, dass es den Durchgang eines Fluids zwischen seinen Maschen gestattet, wobei das verformbare Element an einem Teil seines distalen Endes gehalten wird, wobei das verformbare Element (STN) eine erste Anordnung, die als "Durchgangsanordnung" bezeichnet wird, und eine zweite Anordnung, die als "Halteanordnung" bezeichnet wird, umfasst, wobei der Übergang von der ersten Anordnung zur zweiten Anordnung durch eine Änderung des Durchmessers des verformbaren Elements (STN) erfolgt.

2. Kanülenvorrichtung (1) nach Anspruch 1, **dadurch gekennzeichnet, dass** das verformbare Element (STN) ausdehnbar und zusammenziehbar vermascht ist.

3. Kanülenvorrichtung (1) nach einem der Ansprüche 1 bis 2, **dadurch gekennzeichnet, dass**:
- die erste Anordnung des verformbaren Elements (STN) durch Ausdehnung des verformbaren Elements (STN) erhalten wird, wobei der Durchmesser des verformbaren Elements dann kleiner als eine erste Schwelle ist;
- die zweite Anordnung des verformbaren Elements (STN) seiner Ruhestellungsform entspricht, wobei der Durchmesser des verformbaren Elements dann größer als eine zweite Schwelle ist.

4. Kanülenvorrichtung (1) nach einem der Ansprüche 1 bis 3, **dadurch gekennzeichnet, dass** das verformbare Element (STN) ein "Formgedächtnis" aufweist.

5. Kanülenvorrichtung (1) nach Anspruch 1, **dadurch gekennzeichnet, dass** das verformbare Element ein Stent (STN) ist.

6. Kanülenvorrichtung (1) nach Anspruch 1, **dadurch gekennzeichnet, dass** das verformbare Element (STN) eine Ruhestellungsform umfasst, die einem ersten Haltemittel der Kanülenvorrichtung (1) entspricht, wobei eine der Ruhestellungsformen eine der folgenden Formen sein kann:
- Eine Pilzform;
- Eine Tellerform;
- Eine Trompetenform;
- Eine Ballonform.

7. Kanülenvorrichtung nach Anspruch 1, **dadurch gekennzeichnet, dass** das verformbare Element (STN) die distale Öffnung des Injektionslumens (LI) verlängert, wobei das verformbare Element (STN) ein geschlossenes distales Endstück (11) umfasst.

8. Kanülenvorrichtung nach Anspruch 1, **dadurch gekennzeichnet**, das geschlossene distale Endstück (11) eine stützende Funktion für eine Führungsvorrichtung (12) erfüllt, die in das Injektionslumen (LI) eingeführt ist und einen Anpressdruck auf das distale Endstück (11) ausübt, der in der Lage ist, die Ausdehnung des verformbaren Elements (STN) in längsaxialer Richtung des Injektionslumens (LI) verlängernd zu bewirken.

9. Kanülenvorrichtung nach Anspruch 1, **dadurch gekennzeichnet, dass** das Ansauglumen (LA) und das Injektionslumen (LI) koaxial sind, wobei das Ansauglumen (LA) einen Durchmesser aufweist, der größer ist als der Durchmesser des Injektionslumens (LI).

10. Kanülenvorrichtung nach Anspruch 9, **dadurch gekennzeichnet, dass** das Ansauglumen (LA) eine Zirkulation eines Fluids zwischen der Außenfläche des Injektionslumens (LI) und der Innenfläche des Ansauglumens (LA) über mindestens einen Abschnitt der Kanülenvorrichtung (1) ermöglicht.

11. Kanülenvorrichtung nach Anspruch 10, **dadurch gekennzeichnet, dass** die Verbindung zwischen dem Ansauglumen (LA) und dem Injektionslumen (LI) durch eine allmähliche Verringerung des Durchmessers des Ansauglumens (LA) über mindestens einen Abschnitt hergestellt wird, so dass ein zweites Haltemittel gebildet wird, das die Kanülenvorrichtung (1) in einer Richtung hält, die einer resultierenden Kraft des Haltens der zweiten Anordnung entgegengesetzt ist.

12. Kanülenvorrichtung nach Anspruch 10, **dadurch gekennzeichnet, dass** die Verbindung (13) zwischen dem Ansauglumen (LA) und dem Injektionslumen einen Anschlag bildet, der eine Zunahme des Durchmessers der Kanülenvorrichtung (1) umfasst, die ein drittes Haltemittel bildet, das die Kanülenvorrichtung (1) in einer Richtung entgegengesetzt zu einer resultierenden Kraft des Haltens der zweiten Anordnung hält.

13. Kanülenvorrichtung nach Anspruch 1, **dadurch gekennzeichnet, dass** das Ansauglumen (LA) eine Vielzahl von Öffnungen umfasst, die an seinem Umfang angeordnet sind.

14. Kanülenvorrichtung nach Anspruch 1, **dadurch gekennzeichnet, dass** sie eine Führungsvorrichtung (12) umfasst, die mit dem Endstück (11) des Injektionslumens (LI) zusammenwirkt, um eine Ausdehnung des verformbaren Elements in der die Achse des Injektionslumens (LI) verlängernden Längsrichtung zu bewirken.

15. Künstliche Lunge **dadurch gekennzeichnet, dass** sie Folgendes umfasst:
- eine Kanülenvorrichtung (1) nach einem der Ansprüche 1 bis 14,
- eine Pumpe, die so angeordnet ist, dass sie das Pumpen eines vorgegebenen Fluiddurchflusses gewährleistet, wobei das Ansauglumen (LA) über ein Verbindungsrohr (T1) mit einem Einlass der Pumpe zusammenwirkt;
- einen Oxygenator, der so angeordnet ist, dass er einen Sauerstoffanteil in ein Fluid einführt, wobei ein Einlass des Oxygenators über ein zweites Verbindungsrohr (T2) mit einem Auslass der Pumpe zusammenwirkt;
- einen Wärmetauscher, der so angeordnet ist, dass er den von dem Oxygenator gelieferten Sauerstoff bei einer festgelegten Temperatur in ein Fluidvolumen injiziert, das über ein drittes Verbindungsrohr (T3) in ein Injektionslumen (LI) der Kanülenvorrichtung (1) injiziert wird, wobei der Wärmetauscher über ein viertes Verbindungsrohr (T4) mit dem Oxygenator und der Kanülenvorrichtung (1) verbunden ist.

16. Künstliche Lunge nach Anspruch 15, **dadurch gekennzeichnet, dass** mindestens ein Rohr mit einer Heparinschicht bedeckt ist.

17. Künstliche Lunge nach einem der Ansprüche 15 bis 16, **dadurch gekennzeichnet, dass** die Pumpe so angeordnet und konfiguriert ist, dass sie einen pulsierenden Blutfluss erzeugt.

18. Künstliche Lunge nach einem der Ansprüche 15 bis 17, **dadurch gekennzeichnet, dass** der Oxygenator eine Faserbeschichtung umfasst.

19. Künstliche Lunge nach einem der Ansprüche 15 bis 18, **dadurch gekennzeichnet, dass** der Oxygenator Mittel zur Herstellung einer laminaren Strömung von Sauerstoff umfasst.

## Claims

1. Cannula device (1) for the circulation of a fluid in an artificial lung **characterized in that** it comprises:
- a first lumen (LA), called the aspiration lumen, comprising at least one orifice permitting the aspiration of a volume of the fluid,
- a second lumen (LI), called the injection lumen, comprising at least one orifice permitting the injection of a volume of the fluid,
**characterized in that** the injection lumen (LI) and the aspiration lumen (LA) are held rigidly connected over at least a portion of their length, said lumens being sealed in relation to each other, the injection lumen (LI) comprises at the distal end thereof a deformable element (STN) which is meshed so as to enable the passage of a fluid between its meshes, said deformable element being held over a portion of the distal end thereof, said deformable element (STN) comprising a first setting called "pass" and a second setting called "hold", the change from the first setting to the second setting being effected by a modification of the diameter of said deformable element (STN).

2. Cannula device (1) according to claim 1, **characterized in that** the deformable element (STN) is meshed, extensible and retractable.

3. Cannula device (1) according to any of claims 1 to 2, **characterized in that**:
• the first setting of the deformable element (STN) is obtained by extension of said deformable element (STN), the diameter of the deformable element then being below a first threshold,
• the second setting of the deformable element (STN) is obtained in its rest shape, the diameter of the deformable element then being above a second threshold.

4. Cannula device (1) according to any of claims 1 to 3, **characterized in that** the deformable element (STN) is an element with "shape memory".

5. Cannula device (1) according to claim 1, **characterized in that** the deformable element is a Stent (STN).

6. Cannula device (1) according to claim 1, **characterized in that** the deformable element (STN) comprises a rest shape forming a first means of holding the cannula device (1), one of the rest shapes being able to be one of the following:
• A mushroom shape,
• A plate shape,
• A trumpet shape,
• A balloon shape.

7. Cannula device according to claim 1, **characterized in that** the deformable element (STN) prolongs the distal orifice of the injection lumen (LI), said deformable element (STN) comprising a closed distal tip (11).

8. Cannula device according to claim 1, **characterized in that** the closed distal tip (11) performs a support function for a guiding device (12) introduced into the injection lumen (LI) and exerting a bearing force on said distal tip (11) and being capable of leading to the extension of the deformable element (STN) in the longitudinal direction prolonging the axis of the injection lumen (LI).

9. Cannula device according to claim 1, **characterized in that** the aspiration lumen (LA) and the injection lumen (LI) are coaxial, the aspiration lumen (LA) having a diameter greater than the diameter of the injection lumen (LI).

10. Cannula device according to claim 9, **characterized in that** the aspiration lumen (LA) enables a circulation of a fluid between the outer surface of the injection lumen (LI) and the inner surface of the aspiration lumen (LA) over at least a portion of the cannula device (1).

11. Cannula device according to claim 10, **characterized in that** the junction between the aspiration lumen (LA) and the injection lumen (LI) is formed by a progressive reduction in the diameter of the aspiration lumen (LA) over at least a portion so as to form a second holding means holding the cannula device (1) in a direction opposite to a force resulting from the holding of the second setting.

12. Cannula device according to claim 10, **characterized in that** the junction (13) between the aspiration lumen (LA) and the injection lumen forms a stop comprising an increase in the diameter of the cannula device (1) forming a third holding means holding the cannula device (1) in a direction opposite to a force resulting from the holding of the second setting.

13. Cannula device according to claim 1, **characterized in that** the aspiration lumen (LA) comprises a plurality of orifices arranged on the periphery thereof.

14. Cannula device according to claim 1, **characterized in that** it comprises a guiding device (12) cooperating with the tip (11) of the injection lumen (LI) to cause an extension of the deformable element in the longitudinal direction prolonging the axis of the injection lumen (LI).

15. Artificial lung **characterized in that** it comprises:
• a cannula device (1) according to any of claims 1 to 14,
• a pump arranged to ensure the pumping of a predetermined flow rate of fluid, said aspiration lumen (LA) cooperating with an inlet of the pump via an interface tube (T1),
• an oxygenator arranged to introduce a proportion of oxygen into a fluid, an inlet of the oxygenator cooperating with an outlet of the pump via a second interface tube (T2),
• a heat exchanger arranged to inject oxygen supplied by the oxygenator at a set temperature into a volume of fluid injected into an injection lumen (LI) of the cannula device (1) via a third interface tube (T3), said exchanger being connected to the oxygenator and to the cannula device (1) via a fourth interface tube (T4).

16. Artificial lung according to claim 15, **characterized in that** at least one tube is covered with a layer of heparin.

17. Artificial lung according to any of claims 15 to 16, **characterized in that** the pump is arranged and configured to establish a pulsed flow of blood.

18. Artificial lung according to any of claims 15 to 17, **characterized in that** the oxygenator comprises a fibre coating.

19. Artificial lung according to any of claims 15 to 18, **characterized in that** the oxygenator comprises means for establishing a laminar flow of oxygen.
